# EUROPEAN PATENT APPLICATION

(11) **EP 0 581 136 A1**
(43) Date of publication of application: **02.02.1994**
(21) Application number: 93111407.8
(22) Date of filing: 15.07.1993
(51) Int. Cl.: A61F 13/26, B31F 5/00

(54) **Sleeve for tampon applicator**

(30) Priority: 15.07.1992 DE 4223286
(71) Applicant: McNEIL-PPC, INC., Milltown New Jersey 08850 (US)
(72) Inventor: Schoelling, Hans-Werner, D-58256 Ennepetal (DE); Riediger, Wolfgang Dr, D-40489 Düsseldorf (DE)
(74) Representative: Strehl Schübel-Hopf Groening & Partner

(57) **Abstract**

The invention relates to a sleeve (120) for use as an outer sleeve and/or inner sleeve for a telescopic applicator. The sleeve consists of a blank composed of a plane-surface, rectangular length portion of a flexible, cellulose-containing material. The two ends of the blank are connected to one another to form a joining seam (132) extending in the longitudinal direction of the sleeve (120), the two longitudinal sides of the blank forming the ends of the sleeve (120). At least fore-edges (142, 144) of the two interconnected ends of the blank which are located on the outside of the sleeve (120) are arranged parallel and close against one another and together are aligned with the circumferential surface (140) of the sleeve (120), so that the joining seam (132) is essentially smooth and invisible on the outside of the sleeve (120). As a result of this design, an economical production of the sleeve (120) is achieved and, when the sleeve is used for an applicator for introducing articles into a human or animal body cavity, the danger of irritation or injury to skin tissue is reliably prevented.

## Description

The invention relates to a sleeve for use as an outer sleeve and/or inner sleeve for a telescopic applicator, consisting of a blank composed of a plane-surface, rectangular length portion of a flexible, cellulose-containing material, the two ends of the blank being connected to one another to form a joining seam extending in the longitudinal direction of the sleeve, and the two longitudinal sides of the blank forming the ends of the sleeve.

In sleeves of this generic type, it is known that the joining seam is adhesively bonded in double the thickness of the strip-shaped initial material, such as, for example, cardboard, and leaves behind a non-round inside diameter and a gaping joining seam on the outer circumferential surface of the sleeve. Apart from the fact that the appearance of this sleeve is detrimental in view of the intended purpose mentioned and can prompt psychological reservations as to use, there is also the danger of irritation or injury to the sensitive skin tissue when a sleeve of this type is introduced into a body cavity.

It is known from US-4,522,967 to wind spirally on itself a cardboard layer which is coated with a heat-activatable adhesive. In this, after the first winding, the cardboard strip is folded back on itself and thereafter wound multiply on itself in the opposite direction. The outer end of the coated cardboard strip is located externally in a region situated in front of the radial widening which the material experiences as a result of the first backward folding of the strip at the end of the first winding. This is intended to ensure that the outer end of the coated cardboard strip is inserted into the otherwise cylindrical circumferential surface of the winding sleeve in front of this thickened region. Nonetheless, the edge of the outer end of the cardboard strip is exposed and forms a groove-shaped depression in relation to the adjacent strip material. The appearance of a sleeve of this type is therefore not appreciably improved. Moreover, the possibility of irritation or even injury to the skin tissue by the exposed end edge also cannot be excluded with certainty in this applicator sleeve.

The object on which the invention is based, is, therefore, to improve a sleeve for use as an outer sleeve and/or inner sleeve for a telescopic applicator, especially for a tampon applicator for feminine hygiene, in such a way that, whilst an economical production of the sleeve is ensured, the danger of irritation or injury to skin tissue by the sleeve when it is introduced into and when it is removed from the body cavity is reliably prevented.

The invention achieves this object in that at least fore-edges of the two interconnected ends of the blank which are located on the outside of the sleeve are arranged parallel and close against one another and together are aligned with the circumferential surface of the tubular sleeve, so that the joining seam is essentially smooth and invisible on the outside of the sleeve.

Advantageously, at least parts of the two ends of the blank are designed as bevelled cheeks, the faces of which are directed parallel to one another and are adhesively bonded with one another at a sticking point of the sleeve.

According to the invention, a plurality of embodiments of a sleeve can be developed from a blank consisting of a basic layer and of an outer layer lying above it, the outer layer and the basic layer forming a unit. Thus, the basic layer and the outer layer can be offset relative to one another in the longitudinal direction of the blank such that the one end of the blank is formed by the basic layer and the outer end of the blank by the outer layer.

As the same time, according to one embodiment, there can be provision for ensuring that the ends of the outer layer and of the basic layer are respectively connected to one another by butting flush against one another, so that an outer joining seam of the outer layer and an inner joining seam of the basic layer are formed, with the result that the two joining seams mentioned are offset relative to one another in the circumferential direction of the sleeve.

In a further embodiment of the invention, it is possible that only the ends of the outer layer be connected to one another so as to butt flush against one another and form an outer joining seam, whilst an end portion of the basic layer overlaps its other end and forms with this an inner joining seam which is offset relative to the outer joining seam in the circumferential direction of the sleeve. At the same time, said end portion of the basic layer can be provided with a fold, the other end of the basic layer being located in front of the fold and forming with this the inner joining seam.

In a further embodiment of this sleeve according to the invention, for the production of which is used a blank in which a basic layer and an outer layer likewise form a unit, the basic layer and the outer layer may be made equally long only at one end of the blank, whilst at the other end of the blank the basic layer is made longer than the outer layer. In this case, only the ends of the basic layer are designed as bevelled cheeks which, overlapping, are adhesively bonded with one another, whilst the ends of the outer layer are adhesively bonded with one another so as to butt flush against one another.

Finally, it is also possible, in an embodiment of the sleeve according to the invention which uses a blank produced as a unit from a basic layer and outer layer, that the mutually opposite ends of the basic layer and outer layer of the blank have an equal length and are designed as bevelled cheeks which are directed parallel to one another.

The sleeve according to the invention is especially suitable as an inner sleeve and/or outer sleeve for applicators for the introduction of tampons for feminine hygiene.

It is recommended to use as a material for the blank, sulfate cellulose cardboard which, should the sleeve be used for a tampon applicator for feminine hygiene, advantageously has a weight of 260 g/m² and a thickness of 350 µm. Furthermore, the sulfate cellulose cardboard can be laminated with calcium carbonate and, if appropriate, additionally calendered in a glazing calender. Moreover, a material having as an outer layer varnished paper or a thin plastic foil can also be used for the sleeve blank.

The invention is explained in more detail below by means of the diagrammatic drawing of a plurality of exemplary embodiments. In the drawing:
Figure 1 shows a partially cutaway, perspective view of a blank composed of a rectangular cardboard portion for a first embodiment of a sleeve according to the invention;
Figure 2 shows a view of the blank according to Figure 1, but in which the fore-faces of the two ends of the blank are bevelled to form cheeks;
Figure 3 shows the first embodiment of the sleeve according to the invention, in which the cheeks are connected to one another to form a joining seam;
Figure 4 shows a perspective view of a longitudinally extending, strip-shaped blank for a second embodiment of a sleeve according to the invention, an outer layer being offset relative to a basic layer in the longitudinal direction of the blank;
Figure 5 shows a partially cutaway, perspective view of the second embodiment of the sleeve which is produced from the blank according to Figure 4;
Figure 6 shows a partially cutaway, perspective front and top view of a third embodiment of the sleeve composed of a blank similar to that according to Figure 1;
Figure 7 shows a partially cutaway, perspective representation of a strip-shaped blank having a basic layer and an outer layer for a fourth embodiment of a sleeve according to the invention;
Figure 8 shows the blank according to Figure 7 with parallel cheeks of the basic layer which are bevelled at equal angles;
Figure 9 shows a partially cutaway, perspective view of the fourth embodiment of a sleeve according to the invention produced from the blank according to Figure 8;
Figure 10 shows a cutaway, perspective representation of a blank having a basic layer and an outer layer for a fifth embodiment of the sleeve according to the invention;
Figure 11 shows the blank according to Figure 10 with fore-ends of the basic layer and outer layer of the blank which are bevelled at equal angles; and
Figure 12 shows a cutaway, perspective view of a fifth embodiment of the sleeve according to the invention produced from the blank according to Figure 11.

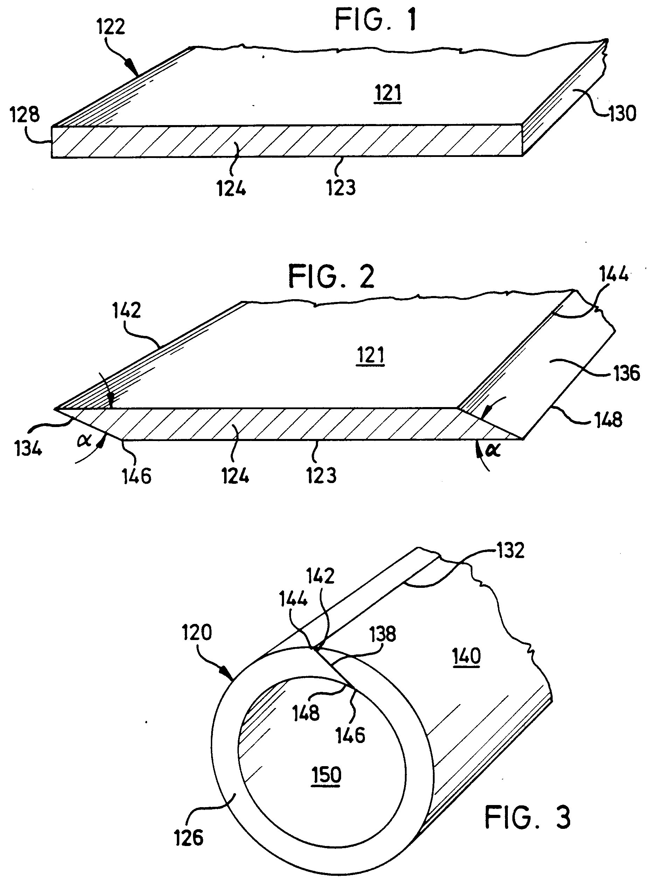

Figure 3 shows a first embodiment of a sleeve 120 for use as an outer sleeve and/or inner sleeve for a telescopic tampon applicator for feminine hygiene. Applicators of this type consist of two sleeves displaceable one in the other, the inner sleeve serving for pushing the tampon out of the outer sleeve. Furthermore, applicators produced with the sleeve according to the invention are also suitable for introducing hygienic and/or medical articles or substances into other body cavities of humans or animals. Applicators of the type mentioned are generally known, and therefore there is no need for a complete representation of them in the drawing.

According to Figure 1, the sleeve 120 consists of a blank 122 composed of a plane-surface, rectangular length portion of a plane-surface, flexible, cellulose-containing material having a topside 121 and an underside 123 parallel to this.

Figure 1 shows a longitudinal edge 124 of the blank 122, the length of which corresponds approximately to the circumferential length of the finished sleeve 120 and which forms an open fore-end 126 of the sleeve 120 in Figure 3. The longitudinal edge of the blank 122, located opposite and parallel to and at a distance from the longitudinal edge 124 and made of equal length, is not shown because of the cutaway representation of the blank 122 in Figures 1 and 2 and of the sleeve 120 in Figure 3. Two mutually parallel ends 128, 130 limiting the length of the blank 122 extend at right angles to the longitudinal edge 124 of the blank 122.

As shown in Figure 2, the fore-faces of the ends 128, 130 of the blank 122 are bevelled, so that they form cheeks 134, 136 which are directed parallel to one another. The angles α of the two cheeks 134, 136 thus correspond to one another, so that, irrespective of the size of the bevel angle, the sleeve 120 preserves the normal thickness of the blank 122 in the region of a joining seam 132. Since the bonding surface made available for the joining seam 132 by the cheeks 134, 136 becomes larger, the smaller the bevel angle selected, the endeavour will be to select the bevel angle of the cheeks 134, 136 as small as possible. Angles of less than 30° are therefore to be preferred. Consequently, the cheeks 134, 136 make in parallel with one another when, according to Figure 3, they are adhesively bonded with one another at a sticking point 138 to form the joining seam 132.

An outer fore-edge 142 and an outer fore-edge 144 of the interconnected ends 128, 130 of the blank 122, which is formed by the two cheeks 134, 136, are arranged parallel and close against one another and together are aligned with an outer circumferential surface 140 of the sleeve 120, so that the joining seam 132 is smooth and virtually invisible on the outside of the sleeve 120.

Correspondingly, an inner fore-edge 146 of one cheek 134 and an inner fore-edge 148 of the other cheek 136 lie so close together in an inner, approximately cylindrical circumferential surface 150 of the sleeve 120 that, there too, a smooth wall is present. Because of the outer and inner, smooth essentially cylindrical circumferential surfaces 140, 150 of the sleeve 120, not only does the latter have a good appearance, but skin irritations or inflammations when the sleeve is introduced into and removed from the body cavity are prevented and an extremely careful handling of an article, such as, for example, a tampon or suppository, arranged in the sleeve is ensured.

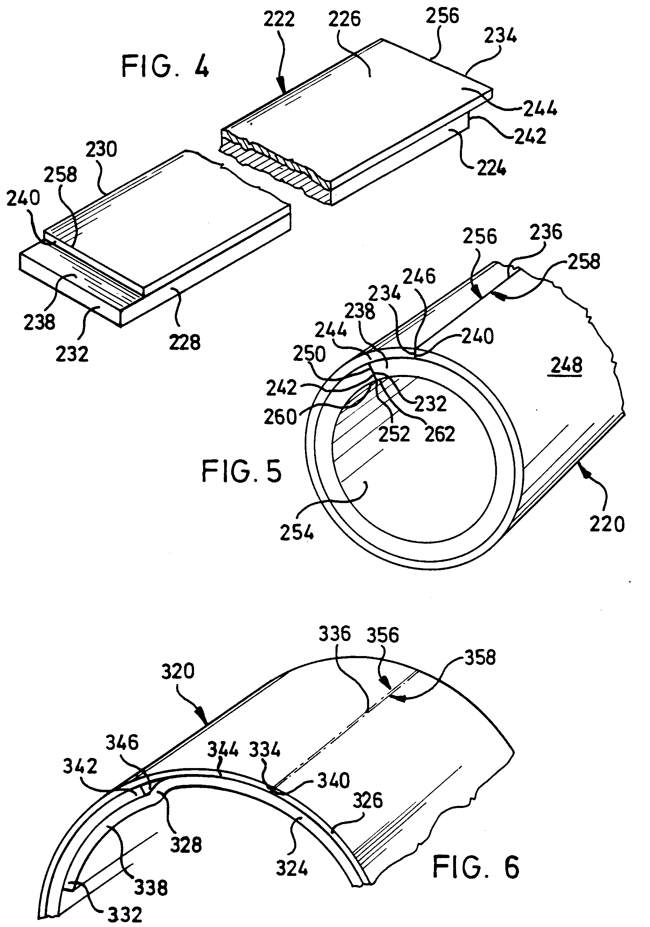

Figure 4 illustrates a blank 222 for a second embodiment of the sleeve 220 in Figure 5. The blank 222, likewise consisting of a plane-surface and rectangular material portion, is composed of a basic layer 224 and of an outer layer 226 which lies above it and which is firmly connected to the basic layer as a unit. Whilst the basic layer 224 and the outer layer 226 are made of equal width so that their longitudinal edges 228, 230 are flush with one another, the outer layer 226 is offset relative to the basic layer 224 in the longitudinal direction of the blank 222, as shown in Figure 4. Consequently, one end 232 of the blank 222 is formed by an end portion 238 of the basic layer 224 which projects beyond an adjacent end 240 of the outer layer 226. Another end 234 of the blank 222 is formed by an end portion 244 of the outer layer 226 which projects beyond an adjacent end 242 of the basic layer 224.

Because the basic layer 224 and the outer layer 226 are offset relative to one another in the longitudinal direction, the end portion 238 of the basic layer 224 and one end portion 244 of the outer layer 226 overlap when a sleeve 220 is formed. As a result, the fore-faces, perpendicular to the main plane of the blank 222 or extending radially relative to the core of the sleeve 220, of the ends 234, 240 of the outer layer 226 lie closely and flush against one another and are connected to one another by means of a sticking point 246, so that a joining seam 236 is formed (Figure 5). At the same time, outer, opposite fore-edges 256, 258 of said ends 234, 240 of the outer layer 226 are aligned with an outer circumferential surface 248 of the sleeve 220 and form the smooth, virtually invisible joining seam 236, as shown in Figure 5. In order to obtain the closed joining seam 236 as a result of a mutual contact of the vertical fore-faces of the ends 234, 240 of the outer layer 226 of the sleeve 220, it is recommended to make the end portion 238 of the basic layer 224 slightly shorter than the end portion 244 of the outer layer 226.

The fore-edges 260, 262 of the two ends 232, 242 of the basic layer 224 are likewise connected to one another by means of a sticking point 250 and form an inner, closed, smooth joining seam 252 which is aligned with an inner, smooth, circumferential surface 254 of the sleeve 220. This sleeve 220 is therefore suitable, like the sleeve of the above-described first embodiment, for receiving a tampon.

Figure 6 shows a third embodiment of the sleeve 320 which can be produced from a blank which is not illustrated, but is similar to that shown in Figure 4. In contrast to the second embodiment, according to Figure 6 one end portion 338 of a basic layer 324 is prefolded radially inwards the amount of the thickness of the basic layer 324 by means of a fold 328. This end portion 338, which has an end 332, therefore overlaps an opposite end 342 of the basic layer 324 and is connected to this by adhesive bonding. The end 342 of the basic layer 324 is located close in front of the fold 328 and forms with the fold 328 an inner joining seam 346.

An outer layer 326 of the sleeve 320 covers the inner joining seam 346 of the basic layer 324. The outer layer 326 projects beyond the end 342 of the basic layer 324 in the circumferential direction of the sleeve 320 by means of an end portion 344 so far that the two opposite ends 334, 340 of the outer layer 326 butt against one another offset relative to the joining seam 346 of the basic layer 324 in the circumferential direction of the sleeve 320 and are adhesively bonded with one another. Outer fore-edges 356, 358 of the ends 334, 340 of the outer layer 326 lie close against one another in the outer, essentially cylindrical circumferential surface of the sleeve 320 and form an outer, smooth joining seam 336 virtually invisible to the eye.

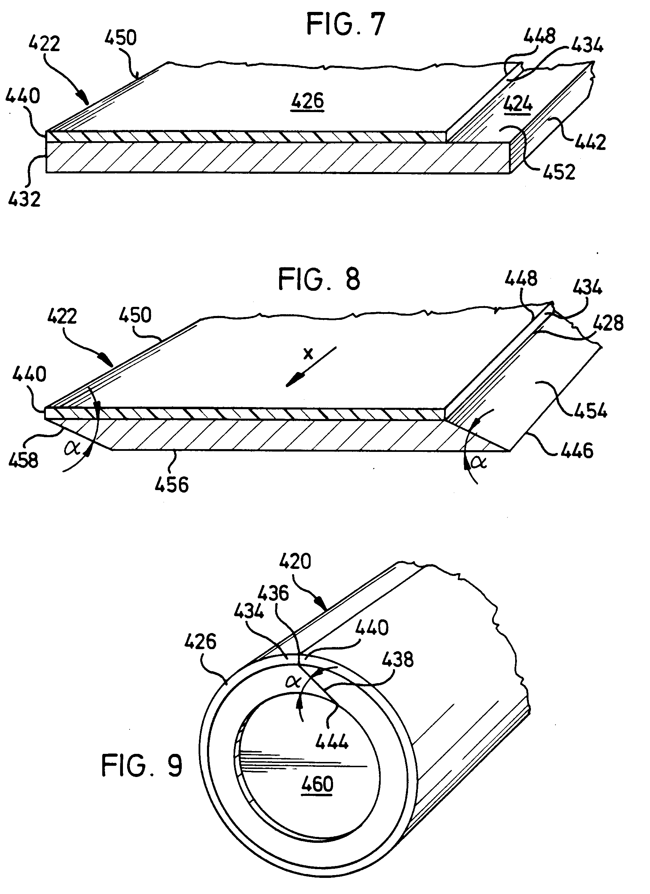

According to Figure 7, in a blank 442 for a fourth embodiment of the sleeve 420 in Figure 9, vertical fore-faces of one end 432 of a basic layer 424 and of one end 440 of an outer layer 426 lie flush one above the other. In contrast, an end portion 452 of the basic layer 424, which has an end 442, projects in the longitudinal direction beyond another end 434 of the outer layer 426. By deformation or machining, for example by grinding or rolling, transversely to the longitudinal direction of the blank 422 in the direction of the arrow x in Figure 8, the projecting end portion 452 of the basic layer 424 is bevelled on the top side facing the outer layer 426 as far as the end 434 of the outer layer 426 and forms a cheek 454. The cheek 454 has a projecting fore-edge 446 which lies in the plane of the underside 456 of the basic layer 424 or of an inner circumferential surface of the sleeve 420. The bevelled cheek 454 projecting beyond the outer layer 426 forms an angle α with the underside 456 of the basic layer 424 and, at its upper or outer end, terminates at a lower end edge 428 of the vertical fore-face of the end 434 of the outer layer 426.

As shown in Figure 8, the end 432 of the basic layer 424 is likewise bevelled by grinding or rolling, so that a cheek 458 corresponding to the cheek 454 is also present at the one end 432 of the basic layer 424, the faces of the cheeks 454, 458 extending parallel to one another. The cheek 458 therefore forms with the underside of the outer layer 426 an angle α, the vertex of which is located at the lower edge of the end 440 of the outer layer 426.

When the blank 442 according to Figure 8 is wound round a winding mandrel 460 in Figure 9 in order to produce a sleeve 420, the cheek 458 at one end of the basic layer 424 overlaps the cheek 454 at the other end of the basic layer 424 and is connected to this at a sticking point 438 to form an inner, closed joining seam 444 which lies in the inner circumferential surface of the sleeve 420 and which is made smooth with this. The ends 434, 440 of the outer layer 426 have outer fore-edges 448, 450 which extend approximately radially relative to the longitudinal mid-axis of the winding mandrel 460, so that, lying close against one another, they form an outer joining seam 436 which closes off the outer surface of the sleeve 420 in a smooth-walled manner. Here too, therefore, the wall thickness of the sleeve 420 corresponds in the region of the joining seams 436, 444 to the thickness of the blank 422.

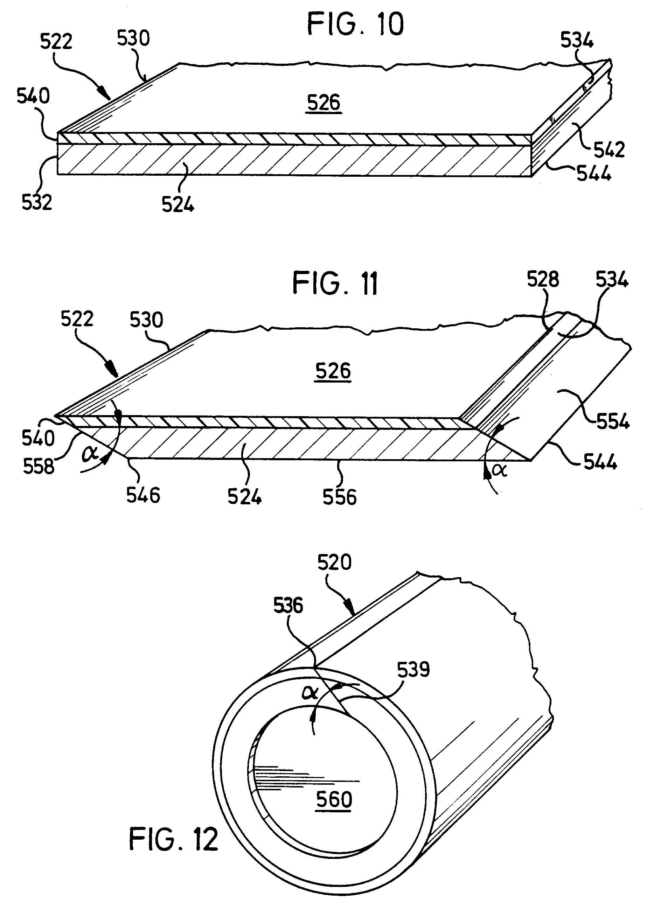

Figure 12 illustrates a fifth embodiment of a sleeve 520 which consists of a rectangular, flat blank 522 in Figure 11, composed of a basic layer 524 and of an outer layer 526 which lie congruently one above the other and which form a fixed unit (Figure 10).

According to Figure 11, in this case not only opposite ends 532, 542 of the basic layer 524, but also opposite ends 534, 540 of the outer layer 526 are respectively bevelled at an equal angle α relative to the topside and underside of the blank 522 by grinding or rolling, so that their angled faces are directed parallel to one another and form respective cheeks 554 and 558. The vertex of the angle α of the cheek 554 coincides with a lower fore-edge 544 of the other fore-end 542 of the basic layer 524. In contrast, the vertex of the angle α of the cheek 558 is located in an upper fore-edge 530 of the fore-end 540 of the outer layer 526. When the bevelled, parallel cheeks 554, 558 are adhesively bonded with one another at a sticking point 539, in this fifth embodiment of the sleeve 520, too, the wall thickness of the latter at the sticking point 539 corresponds to the wall thickness of the sleeve wall or the blank 522. At the same time, outer fore-edges 528, 530 of the cheeks 554, 558 once again lie closely flush against one another in the outer circumferential surface of the sleeve 520, so that a smooth joining seam 536 which is virtually invisible is obtained on the circumferential surface of the sleeve.

In the present exemplary embodiment, however, inner fore-edges 544, 546 of the cheeks 554, 558 also lie close to one another in the inner, essentially cylindrical circumferential surface of the sleeve 520. Consequently, the single, inwardly continuous joining seam 536 is formed by said bevelled cheeks 554, 558 (Figure 12) when the blank 522 is wrapped round a winding mandrel 560 and the cheeks 554, 558 are overlapped and adhesively bonded, as described. As the joining seam 536 lies both internally and externally in the inner and outer circumferential surface of the sleeve 520 respectively, this sleeve 520 is also suitable especially as an outer sleeve for a tampon applicator for feminine hygiene.

A sulfate cellulose cardboard in particular is suitable as a basic material for producing the above-described sleeves. If the sleeves are used for tampon applicators for feminine hygiene, it is recommended to use a sulfate cellulose cardboard of a weight of approximately 260 g/m² and of a thickness of approximately 350 µm. Unevenesses of the material can be laminated with calcium carbonate. Furthermore, a glossy appearance can be imparted to the surface of the material by means of a glazing calender. The above-described first embodiment of the sleeve 120 can be produced completely from the material mentioned. It dissolves easily in water and, if appropriate, is also reusable. It is also possible to use sulfate cellulose cardboard for the four last-mentioned embodiments of the sleeve according to the invention. In these cases, the outer layer of the sleeve can also consist of other materials, such as, for example, a foil composed of a preferably biologically degradable plastic, varnished paper or the like. The blank can consist not only partially, but also completely of biologically degradable plastic.

For the adhesive bonding of the ends of the blank to form the sleeve, conventional, hot-melt adhesives, which are generally known to an average person skilled in the art and which are therefore not listed in detail, are preferred.

### List of Reference Symbols

- 120: Sleeve
- 121: Top side
- 122: Blank
- 123: Underside
- 124: Longitudinal edge
- 126: Open fore-end
- 128,130: Ends of the blank
- 132: Joining seam
- 134,136: Cheeks
- 138: Sticking point
- 140: Outer circumferential surface
- 142,144: Outer fore-edges
- 146,148: Inner fore-edges
- 150: Inner circumferential surface
- 220: Sleeve
- 222: Blank
- 224: Basic layer
- 226: Outer layer
- 228: Longitudinal edge
- 230: Longitudinal edge
- 232: End
- 234: End
- 236: Joining seam
- 238: End portion
- 240: End
- 242: End
- 244: End portion
- 246: Sticking point
- 248: Outer circumferential surface
- 250: Sticking point
- 252: Inner joining seam
- 254: Inner circumferential surface
- 256,258: Outer fore-edges
- 260,262: Fore-edges
- 320: Sleeve
- 322:
- 324: Basic layer
- 326: Outer layer
- 328: Fold
- 330:
- 332: End
- 334: End of outer layer
- 336: Joining seam of outer layer
- 338: End portion
- 340: End of outer layer
- 342: End of basic layer
- 344: End portion
- 346: Inner joining seam
- 348:
- 350:
- 352:
- 354:
- 356: Fore-edge
- 358: Fore-edge
- 420: Sleeve
- 422: Blank
- 424: Basic layer
- 426: Outer layer
- 428: Lower end edge
- 430:
- 432: End
- 434: End of outer layer
- 436: Joining seam
- 438: Sticking point
- 439: Inner joining seam
- 440: End of outer layer
- 442: End
- 444: Inner joining seam
- 446: Fore-edge
- 448: Outer fore-edge
- 450: Outer fore-edge
- 452: Basic layer
- 454: Cheek
- 456: Underside
- 458: Cheek
- 460: Winding mandrel
- 520: Sleeve
- 522: Blank
- 524: Basic layer
- 526: Outer layer
- 528:
- 530: Upper fore-edge
- 532: End of basic layer
- 534: End of outer layer
- 536: Joining seam
- 538:
- 539: Sticking point
- 540: End of outer layer
- 542: End of basic layer
- 544: Lower fore-edge
- 546: Inner fore-edge
- 548:
- 550:
- 552:
- 554: Cheek
- 556:
- 558: Cheek
- 560: Winding mandrel

## Claims

1. Sleeve for use as an outer sleeve and/or inner sleeve for a telescopic applicator, consisting of a blank composed of a plane-surface, rectangular length portion of a flexible, cellulose-containing material, the two ends of the blank being connected to one another to form a joining seam extending in the longitudinal direction of the sleeve, and the two longitudinal sides of the blank forming the ends of the sleeve, characterised in that at least fore-edges (142, 144; 256, 258; 356, 358; 448, 450; 528, 530) of the two interconnected ends (128, 130; 232, 234; 240, 242; 332, 334; 340, 342; 434, 440, 454, 458; 534, 540, 554, 558) of the blank (122; 222; 322; 422; 522) which are located on the outside of the sleeve (120; 220; 320; 420; 520) are arranged parallel and close against one another and together are aligned with the circumferential surface of the tubular sleeve (120; 220; 320; 420; 520), so that the joining seam (132; 236; 336; 436; 536) is essentially smooth and invisible on the outside of the sleeve (120; 220; 320; 420; 520).

2. Sleeve according to Claim 1, characterised in that at least parts of the two ends of the blank (122; 422; 522) are designed as bevelled cheeks (134, 136; 454, 458; 534, 540, 554, 558), the faces of which are directed parallel to one another and are adhesively bonded with one another at a sticking point (138; 444; 539) of the sleeve (120; 420; 520).

3. Sleeve according to Claim 1, consisting of a blank which is composed of a basic layer and of an outer layer lying above it, the outer layer and the basic layer forming a unit, characterised in that the basic layer (224; 324) and the outer layer (226; 326) of the blank (222; 322) are offset relative to one another in the longitudinal direction of the latter such that the one end (232; 332) of the blank (222; 322) is formed by the basic layer (224; 324) and the other (234; 334) of the blank by the outer layer (226; 326).

4. Sleeve according to Claim 3, characterised in that the ends (234, 240) of the outer layer (226) are connected so as to butt flush against one another and form an outer joining seam (236), and in that the ends (232, 242) of the basic layer (224) are connected so as to butt flush against one another, so that an outer joining seam (236) of the outer layer (226) and an inner joining seam (252) of the basic layer (224) are offset relative to one another in the circumferential direction of the sleeve (220).

5. Sleeve according to Claim 3, characterised in that only the ends (334, 340) of the outer layer (326) are connected to one another so as to butt flush against one another and form an outer joining seam (336), whilst an end portion (338) of the basic layer (324) overlaps its other end (342) and forms with this the inner joining seam (346) which is offset relative to the outer joining seam (336) in the circumferential direction of the sleeve (320).

6. Sleeve according to Claim 5, characterised in that the end portion (338) of the basic layer (324) is provided with a fold (328), the other end (342) of the basic layer (324) being located in front of the fold (328) and forming with this the inner joining seam (328).

7. Sleeve according to Claim 1 or 2, which consists of a blank composed of a basic layer and of an outer layer which form a unit, characterised in that the basic layer (424) and the outer layer (426) are made equally long only at one end (432, 440) of the blank (422), whilst at the other end (434, 442) of the blank (422) the basic layer (424) is made longer than the outer layer (426), and in that only the ends (432, 442) of the basic layer (424) are designed as bevelled cheeks (454, 458) which, overlapping, are adhesively bonded with one another, whilst the ends (434, 440) of the outer layer (426) are adhesively bonded with one another so as to butt flush against one another.

8. Sleeve according to Claim 1 or 2, consisting of a blank composed of a basic layer and of an outer layer which form a unit, characterised in that the mutually opposite ends (532, 540; 534, 542) of the basic layer (524) and outer layer (526) of the blank (522) have an equal length and width and are designed as bevelled cheeks (534, 540, 554, 558) which are directed parallel to one another.

9. Sleeve according to one of Claims 1 to 8, characterised by its use as an inner sleeve and/or as an outer sleeve for an applicator for the introduction of tampons for feminine hygiene.

10. Sleeve according to one of Claims 1 to 9, characterised in that the blank consists of sulphate cellulose cardboard.

11. Sleeve according to Claim 10, characterised in that the sulphate cellulose cardboard has a weight of 260 g/m² and a thickness of 350 m.

12. Sleeve according to Claim 10 or 11, characterised in that the sulphate cellulose cardboard is laminated with calcium carbonate.

13. Sleeve according to one of Claims 10 to 12, characterised in that the sulphate cellulose cardboard is calendered in a glazing calender.

14. Sleeve according to one of Claims 1 to 13, characterised in that the blank consists of a laminate, the outer layer consisting of varnished paper.

15. Sleeve according to one of Claims 1 to 13, characterised in that the blank consists of a laminate, the outer layer consisting of a plastic foil.

16. Sleeve according to one of Claims 1 to 15, characterised in that the blank consists at least partially of biologically degradable plastic.
